# EUROPÄISCHE PATENTANMELDUNG

(11) **EP 4 700 698 A1**
(43) Veröffentlichungstag der Anmeldung: **25.02.2026**
(21) Anmeldenummer: 25195666.0
(22) Anmeldetag: 13.08.2025
(51) Int. Cl.: G06T 7/00, G01N 21/90

(54) **VERFAHREN ZUR DURCHFÜHRUNG EINER BEHÄLTNISINSPEKTIONSAUFGABE IN EINER BEHÄLTNISBEHANDLUNGSANLAGE UND BEHÄLTNISINSPEKTIONSVORRICHTUNG FÜR EINE BEHÄLTNISBEHANDLUNGSANLAGE**

(30) Priorität: 22.08.2024 DE 102024124076
(71) Anmelder: Krones AG, 93073 Neutraubling (DE)
(72) Erfinder: Hewicker, Alexander, 93073 Neutraubling (DE); Meierhofer, Max, 93073 Neutraubling (DE)
(74) Vertreter: Bittner, Bernhard

(57) **Zusammenfassung**

Verfahren zur Durchführung einer Behältnisinspektionsaufgabe in einer Behältnisbehandlungsanlage zur Behandlung einer Vielzahl von Behältnisteilen für Behältnisse, bevorzugt Kunststoffbehältnisse und/oder Flaschen, in welcher eine Transporteinrichtung, die Vielzahl von Behältnisteilen als Behältnisteil-Strom entlang eines vorgegebenen Transportpfads von wenigstens einer Behandlungseinrichtung der Behältnisbehandlungsanlage zu wenigstens einer weiteren Behandlungseinrichtung der Behältnisbehandlungsanlage transportiert und wenigstens eine Sensoreinrichtung zur Durchführung der Behältnisinspektionsaufgabe insbesondere ortsaufgelöste Sensordaten, bevorzugt Kamerabilder, in Bezug auf die zu inspizierenden Behältnisteile, bevorzugt optisch, erfasst und eine Echtzeit-Auswertungseinrichtung die insbesondere ortsaufgelösten Sensordaten, bevorzugt die Kamerabilder, in Echtzeit auswertet. Erfindungsgemäß ist der Echtzeit-Auswertungseinrichtung ein Satz von Behältnisteil-Merkmalen vorgegeben, bei welchem es sich um einen Satz von, im Rahmen eines in Bezug auf eine von der Behältnisinspektionsaufgabe verschiedenen Trainings-Behältnisinspektionsaufgabe durchgeführten maschinellen Lernverfahrens automatisch, insbesondere von einem neuronalen Netz, extrahierten Behältnisteil-Merkmalen handelt, und dass die Echtzeit-Auswertungseinrichtung die Behältnisinspektionsaufgabe basierend auf dem vorgegebenen Satz von Behältnisteil-Merkmalen durchführt.

## Beschreibung

Die vorliegende Erfindung bezieht sich auf ein Verfahren zur Durchführung einer Behältnisinspektionsaufgabe in einer Behältnisbehandlungsanlage zur Behandlung einer Vielzahl von Behältnisteilen (etwa das Behältnis selbst oder ein Behältnisverschluss) für Behältnisse, bevorzugt Kunststoffbehältnisse und/oder Flaschen. Dabei transportiert in der Behältnisbehandlungsanlage eine Transporteinrichtung die Vielzahl von (zu inspizierenden) Behältnisteilen als Behältnisteil-Strom entlang eines vorgegebenen Transportpfads von wenigstens einer (ersten) Behandlungseinrichtung der Behältnisbehandlungsanlage zu wenigstens einer weiteren Behandlungseinrichtung der Behältnisbehandlungsanlage.

Weiter erfasst wenigstens eine Sensoreinrichtung zur Durchführung der Behältnisinspektionsaufgabe, insbesondere ortsaufgelöste, Sensordaten wie etwa Kamerabilder in Bezug auf die zu inspizierenden Behältnisteile. Diese erfassten Sensordaten werden insbesondere in Echtzeit von einer Echtzeit-Auswertungsvorrichtung ausgewertet.

Die vorliegende Erfindung bezieht sich weiter auf eine Behältnisinspektionsvorrichtung für eine Behältnisbehandlungsanlage sowie ein Verfahren zur Ermittlung von Behältnisteil-Merkmalen.

Bei den Behältnissen handelt es sich bevorzugt um Kunststoffbehältnisse (insbesondere PET-Behältnisse), Behältnisse deren Hauptbestandteil aus Pulpe besteht und/oder Glasbehältnisse und/oder Dosen. Bei den Behältnissen kann es sich dabei um Behältnisse aus der Getränke- und/oder Lebensmittel- und/oder Kosmetikindustrie handeln. Beispielsweise kann es sich um Dosen oder Flaschen, wie etwa Glasflaschen, Pulpeflaschen und Kunststoffflaschen handeln.

In Behälterbehandlungsanlagen, etwa Behälter-Abfüllanlagen, kommen beispielsweise zur Prozesskontrolle verschiedenste Sensoriken und Bildverarbeitungssysteme zum Einsatz. Für verschiedene Prozessschritte wie Spritzgießen, Behältnisreinigung, Abfüllen, Etikettieren Verschließen, Einpacken, Umreifen und/oder Schrumpfverpacken erfolgt im Anschluss beispielsweise eine optische Inspektion zur Kontrolle und/oder zur Steuerung oder Regelung eines Prozesses.

Für jeden Prozessschritt werden üblicherweise Kontrollsysteme, meist Bildverarbeitungssysteme, installiert, welche zu konfigurieren und aufwändig zu parametrieren sind. Dies erfordert viel Erfahrung und Fingerspitzengefühl.

Momentan werden in der Inspektionstechnik zwei Arten von Bild-Klassifikatoren eingesetzt.

Erstens sind klassische Klassifikationsverfahren bekannt, die auf wenigen, aus dem Bild extrahierten Merkmalen, eine Klassifikation mit Algorithmen wie Support-Vektor-Machines, k-Nearest-Neighbour oder Entscheidungs-Bäumen ausführen.

Zweitens sind moderne, Kl-basierte (Künstliche Intelligenz-basierte) Klassifikationverfahren bekannt, die zumeist auf einem neuronalen Netz basieren. Häufig wird das neuronale Netz in der Ausprägung als convolutional neural network verwendet. Die Klassifikatoren beinhalten bereits eine gelernte Merkmalsextraktion, die besonders gut funktioniert, aber unveränderlich ist.

Klassische Klassifikationsverfahren benötigen eine vorgelagerte Merkmalsextraktion, die die Daten aus dem Bild auf wenige extrahierte Merkmale reduziert. Hierbei werden die Merkmale häufig "von Hand" ausgewählt und extrahiert. Die Entscheidungsqualität des Klassifikators ist auf Grund der starken Reduzierung der Daten und der begrenzten Leistungsfähigkeit der Klassifikation-Algorithmen stark begrenzt.

Moderne KI-Klassifikatoren lernen die Extraktion der relevanten Merkmale aus dem Bild und passen sich durch den Lernvorgang sehr gut an die Aufgabenstellung an. Sie erreichen bei der Bild-Klassifikation eine hervorragende Entscheidungsqualität. Nachteilig ist allerdings der lange Lernvorgang und der hohe Bedarf an Lerndaten. Dies macht eine schnelle Anpassung an veränderte Aufgabenstellungen (z.B. neue Flaschensorten im Bild) unmöglich.

Der vorliegenden Erfindung liegt die Aufgabe zugrunde, die aus dem Stand der Technik bekannten Nachteile zu überwinden und ein Verfahren zum Durchführen einer Behältnisinspektionsaufgabe in einer Behältnisbehandlungsanlage sowie eine Behältnisinspektionsvorrichtung für eine Behältnisbehandlungsanlage bereitzustellen, welche an neue Inspektionsaufgaben schnell anpassbar ist und gleichermaßen eine sehr hohe Inspektionsleistung bietet.

Die Aufgabe wird erfindungsgemäß durch die Gegenstände der unabhängigen Ansprüche gelöst. Vorteilhafte Ausführungsformen und Weiterbildungen der Erfindung sind Gegenstand der Unteransprüche.

Bei einem erfindungsgemäßen Verfahren zur Durchführung einer Behältnisinspektionsaufgabe in einer Behältnisbehandlungsanlage zur Behandlung einer Vielzahl von Behältnisteilen für Behältnisse, bevorzugt Kunststoffbehältnisse und/oder Flaschen, transportiert in der Behältnisbehandlungsanlage eine Transporteinrichtung die (zu behandelnde und/oder behandelte) Vielzahl von (zu inspizierenden) Behältnisteilen als Behältnisteil-Strom entlang eines vorgegebenen Transportpfads von wenigstens einer (ersten) Behandlungseinrichtung der Behältnisbehandlungsanlage zu wenigstens einer weiteren Behandlungseinrichtung der Behältnisbehandlungsanlage.

Bei den Behältnissen handelt es sich bevorzugt um Kunststoffbehältnisse (insbesondere PET-Behältnisse), Behältnisse deren Hauptbestandteil aus Pulpe besteht und/oder Glasbehältnisse und/oder Dosen. Bei den Behältnissen kann es sich dabei um Behältnisse aus der Getränke- und/oder Lebensmittel- und/oder Kosmetikindustrie und/oder Pharmaindustrie handeln. Beispielsweise kann es sich um Dosen oder Flaschen, wie etwa Glasflaschen, Pulpeflaschen und Kunststoffflaschen handeln.

Unter einem "Behältnisteil für ein Behältnis" kann auch das Behältnis selbst verstanden werden. So könnte es sich bei dem Behältnisteil etwa um einen Vorformling handeln, aus welchem durch einen Umformvorgang das fertig ausgeformte Behältnis hergestellt wird, oder um das bereits fertig ausgeformte Behältnis handeln.

Bei dem Behältnisteil für ein Behältnis kann es sich auch um eine Ausstattung des Behältnisses, wie ein (bevorzugt wiederverschließbarer) Behältnisverschluss (etwa Drehverschluss oder Kappe), ein (PET- und/oder Kunststoff-)Deckel, ein Etikett, eine (Laser- oder Direktdruck- )Markierung, ein Füllgut und/oder eine Verpackung des (fertigen) Behältnisses, eine Behältniszusammenstellung oder dergleichen (sowie Kombinationen hiervon) handeln.

So kann es sich beispielsweise bei der Transporteinrichtung um eine Zuführung (etwa eine Zuführschiene, für Behältnisverschlüsse handeln, welche die Behältnisverschlüsse ausgehend von einer Sammeleinrichtung einer Verschließeinrichtung zum Verschließen von Behältnissen zuführt.

Bevorzugt handelt es sich bei dem Behältnisteil um einen von der Transporteinrichtung als (genau eine) Einheit transportablen und (unabhängig von weiteren transportierten Behältnisteilen) transportierten Gegenstand.

Bevorzugt führt die (erste) Behandlungseinrichtung und/oder die (wenigstens eine) weitere Behandlungseinrichtung (insbesondere jeweils) wenigstens einen Behandlungsschritt an dem Behältnisteil (und bevorzugt an dem Behältnis) durch.

Der Behandlungsschritt der (ersten) Behandlungseinrichtung und/oder der (wenigstens einen) weiteren Behandlungseinrichtung kann ausgewählt sein aus einer Gruppe von Behandlungsschritten, welche einen Spritzgießvorgang zum Herstellen eines Spritzgießteils (etwa Kunststoffvorformling), einen Reinigungsvorgang, einen Zerkleinerungsvorgang und/oder Zerteilungsvorgang (etwa im Rahmen eines Recyclingvorgangs), einen Umformvorgang (insbesondere ein (Streck-)Blasformen), einen (Laser-)Markierungsvorgang, einen Individualisierungsvorgang (etwa Anbringen eines QR-Codes), einen Ausleitvorgang (in welchem das Behältnisteil aus dem Behältnisteil-Strom der Behältnisbehandlungsanlage ausgeleitet wird), einen Sortiervorgang (in welchem die Behältnisteile sortenabhängig sortiert werden), einen Füllvorgang, einen Verschließvorgang, einen (insbesondere Direkt-)Druckvorgang, einen Etikettiervorgang, ein Vornehmen einer Laserdekoraktion, einen Verpackungsvorgang (insbesondere ein Abringen einer Primär- und/oder Sekundärverpackung, etwa Einschweißen mehrerer Behältnisse als Paket und/oder als Gebindeeinheit), eine Bestimmung der Zusammensetzung von Stoffen oder Stoffgemischen, etwa der Atmosphäre und/oder Luft in oder an einem Behältnisteil und bevorzugt Behältnis, beispielsweise mittels eines Massenspektrometers und/oder einer Geruchssensoreinrichtung, und dergleichen sowie Kombinationen hiervon umfasst.

Weiter erfasst wenigstens eine Sensoreinrichtung zur (bevorzugt Behältnisteil-individuellen) Durchführung der Behältnisinspektionsaufgabe, insbesondere ortsaufgelöste, Sensordaten in Bezug auf die zu inspizierenden Behältnisteile.

Bevorzugt wird das zu inspizierende Behältnisteil wenigstens bereichsweise und bevorzugt dessen aus wenigstens einer Beobachtungsrichtung beobachtbarer bzw. sichtbarer Behältnisteilbereich in den Sensordaten abgebildet.

Denkbar ist, dass zu jedem zu inspizierenden Behältnisteil einzeln bzw. Behältnisteil-individuell Sensordaten erfasst bzw. erhoben werden (in jeweils einem eigenen Erfassungsschritt der Sensoreinrichtung).

Es kann genau eine Sensoreinrichtung vorgesehen sein, welche die zur Durchführung der Behältnisinspektionsaufgabe erforderlichen Sensordaten in Bezug auf die Behältnisteile erfasst. Denkbar ist aber auch, dass hierfür mehrere Sensoreinrichtungen vorgesehen ist, welche beispielsweise die Sensordaten aus mehreren Aufnahmerichtungen in Bezug auf das Behältnisteil erfassen und/oder welche in einem mehrbahnigen Transportbereich jeweils die auf verschiedenen Bahnen transportierten Behältnisteile erfassen.

Bei den Sensordaten handelt es sich bevorzugt um ortsaufgelöste Sensordaten, welche insbesondere eine zu erfassende Eigenschaft (etwa Farbwert und/oder Grauwert und/oder Helligkeitswert) eines Bereichs des Behältnisteils abbilden. Bevorzugt geben die ortsaufgelösten Sensordaten einen Sensordatenverlauf in Abhängigkeit von wenigstens einer räumlichen und/oder geometrischen Koordinate und bevorzugt in Abhängigkeit von wenigstens zwei räumlichen und/oder geometrischen Koordinaten an (oder sind angebbar).

Bei den Sensordaten kann es sich - wie etwa bei den von einer Kamera erfassten Sensordaten - etwa um einen Farbwert und/oder Grauwert und/oder Helligkeitswert handeln.

Bei den von einer LIDAR-Einrichtung erfassten Sensordaten kann es sich um RGB-Werte und/oder Intensitätswerte handeln, welche für jeden erfassten Datenpunkt zusammen mit bzw. in Abhängigkeit von dessen X-, Y- und Z-Positionswert erfasst und abgelegt werden.

Denkbar ist auch, dass es sich bei den Sensordaten um frequenzaufgelöste Sensordaten handelt.

So kann etwa je Sensordatenpunkt wenigstens ein Intensitätswert in Abhängigkeit einer Frequenz der von der Sensoreinrichtung erfassten Strahlung erfasst werden, so dass die Sensordaten einen Sensorwertverlauf in Abhängigkeit einer Frequenz angeben.

Denkbar ist auch, dass es sich bei den Sensordaten um Spektrometer-Sensordaten handelt, welche bevorzugt von einem Massenspektrometer, welches etwa als Geruchssensor verwendet wird, erzeugt werden. Denkbar ist, dass damit etwa eine Zusammensetzung eines Gases und/oder Luft und/oder Luftgemischs, etwa einer Atmosphäre in einem Behältnis analysiert wird. Hier können etwa die erfassten Sensordaten einen Intensitätswertverlauf in Abhängigkeit eines Masse-zu-Ladung-Verhältnisses von in der Atmosphäre enthaltenen Atomen und Molekülen angeben.

Bevorzugt handelt es sich bei der Erfassung der insbesondere ortsaufgelösten Sensordaten um eine optische Erfassung. Bevorzugt handelt es sich bei den ortsaufgelösten Sensordaten um Kamerabilder.

Bevorzugt werden die Sensordaten in Bezug auf die zu inspizierenden Behältnisteile während des Transports dieser jeweils zu erfassenden Behältnisteile mit unveränderter insbesondere nicht reduzierter Transportgeschwindigkeit erfasst, also während sich die Behältnisteile in Bewegung befinden. Zur Erfassung der Sensordaten werden mit anderen Worten die Behältnisteile nicht abgebremst und/oder angehalten. Dies bietet den Vorteil einer hohen Durchlaufgeschwindigkeit und Produktionsgeschwindigkeit der Behältnisbehandlungsanlage.

Zur optischen Erfassung der Sensordaten kann eine Beleuchtung der Behältnisteile und bevorzugt Behältnisse vorgesehen sein, etwa eine Auflicht- und/oder eine Durchlichtbeleuchtung.

Weiter wertet eine, insbesondere Prozessor-basierte, Echtzeit-Auswertungseinrichtung die (erfassten), insbesondere ortsaufgelösten, Sensordaten, bevorzugt die Kamerabilder, in Echtzeit aus (insbesondere im Rahmen eines Computer-implementierten Verfahrensschritts bzw. als Computer-implementierter Verfahrensschritt).

Dabei erfolgt bevorzugt auch hier die Auswertung der Sensordaten Behältnisteil-individuell, also insbesondere jeweils separat in Bezug auf einzelne Behältnisteile.

Erfindungsgemäß ist und/oder wird der Echtzeit-Auswertungseinrichtung ein Satz von Behältnisteil-Merkmalen vorgegeben und/oder bereitgestellt, bei welchem es sich um einen Satz von, im Rahmen eines in Bezug auf eine Trainings-Behältnisinspektionsaufgabe durchgeführten maschinellen Lernverfahrens automatisch, insbesondere von einem neuronalen Netz, extrahierten Behältnisteil-Merkmalen handelt. Dabei ist bevorzugt die Trainings-Behältnisinspektionsaufgabe von der Behältnisinspektionsaufgabe verschieden. Die Behältnisteil-Merkmale werden bevorzugt bei einem maschinellen Lernverfahren bzw. bei Vornahme eines maschinellen Lernverfahrens extrahiert, welches in Bezug auf eine Trainings-Behältnisinspektionsaufgabe durchgeführt wird. Mit der Durchführung des maschinellen Lernverfahrens soll ein (trainierter) Algorithmus bzw. ein (trainiertes) Modell (maschinellen Lernens) erhalten werden, welches zur Erfüllung bzw. zur Durchführung der Trainings-Behältnisinspektionsaufgabe dient.

Bevorzugt handelt es sich bei dem Satz von extrahierten Behältnisteil-Merkmalen um einen, im Rahmen des in Bezug auf die Trainings-Behältnisinspektionsaufgabe durchgeführten maschinellen Lernverfahrens (automatisch) extrahierten Satz von (extrahierten) Behältnisteil-Merkmalen.

Der bereitgestellte Satz von Behältnisteil-Merkmalen wird bevorzugt durch Übermittlung von einem (in Bezug auf die Behältnisbehandlungsanlage bzw. Bedieners der Behältnisbehandlungsanlage) externen Server und/oder einen externen Speichereinrichtung (etwa eines Herstellers der Behältnisbehandlungsanlage) empfangen. Dabei erfolgt die Übermittlung wenigstens abschnittsweise über nicht-private Kommunikationsverbindungen.

Weiter führt die Echtzeit-Auswertungseinrichtung die Behältnisinspektionsaufgabe basierend auf dem vorgegebenen Satz von Behältnisteil-Merkmalen durch (im Rahmen eines Computer-implementierten Verfahrensschritts). Die Echtzeit-Auswertungseinrichtung bewertet bevorzugt die in Bezug auf (bevorzugt genau) ein zu inspizierendes Behältnisteil erfassten Sensordaten auf Grundlage des vorgegebenen Satzes von Behältnisteil-Merkmalen.

Bevorzugt ermittelt die Echtzeit-Auswertungseinrichtung in Abhängigkeit des vorgegebenen und/oder bereitgestellten Satzes von Behältnisteil-Merkmalen, ob (wenigstens) eines dieser Behältnisteil-Merkmale (zu einer vorgegebenen Wahrscheinlichkeit) in den Sensordaten abgebildet bzw. gezeigt ist.

Bevorzugt ermittelt die Echtzeit-Auswertungseinrichtung in Abhängigkeit der Auswertung/Bewertung ein Inspektionsergebnis zu der durchzuführenden Behältnisinspektionsaufgabe. Bevorzugt erfolgt in Abhängigkeit des ermittelten Inspektionsergebnisses eine Steuerung und/oder Regelung wenigstens eines Behandlungs- und/oder Produktionsprozesses der Behältnisbehandlungsanlage. Denkbar ist, dass in Abhängigkeit des Inspektionsergebnisses (etwa von der Behältnisbehandlungsanlage und/oder einer Behandlungseinrichtung) ermittelt wird, ob das inspizierte Behältnisteil auszuleiten ist. Bevorzugt wird bei einem als auszuleitend bewerteten Behältnisteil eine Ausleitung vorgenommen.

Bevorzugt sind die wenigstens eine Sensoreinrichtung und die Echtzeit-Auswertungseinrichtung Bestandteil einer (nachfolgend nähere beschriebenen) Behältnisinspektionsvorrichtung (für eine/die Behältnisbehandlungsanlage).

Eine (jeweilige) Sensoreinrichtung kann dabei nach jedem Behandlungsschritt Sensordaten erfassen, um das Behandlungsergebnis zu überprüfen. Hierzu kann die Behältnisbehandlungsanlage eine Vielzahl von derartigen Sensoreinrichtungen aufweisen.

Mit anderen Worten wird vorgeschlagen, einen mittels Kl-basierter Merkmalsextraktion erhaltenen Satz von extrahierten Behältnisteil-Merkmalen für (wenigstens) eine Behältnisinspektonsaufgabe zu verwenden, welche bei der Kl-basierten Merkmalsextraktion nicht berücksichtigt wurde. Während also die KI-basierte Merkmalsextraktion während oder im Rahmen eines maschinellen Lernverfahrens zur Durchführung der Trainings-Behältnisinspektionsaufgabe erfolgte und damit die zur Trainings-Behältnisinspektionsaufgabe wesentlichsten Merkmale angibt, wird keine eigene spezifische Merkmalsextraktion für die Behältnisinspektionsaufgabe durchgeführt, sondern der zur Trainings-Behältnisinspektionsaufgabe erhaltene Satz extrahierter Behältnisteil-Merkmale bei der Durchführung der Behältnisinspektionsaufgabe verwendet.

Dies bietet den Vorteil, dass durch den Verzicht auf eine neuerliche Merkmalsanalyse ein zeit- und ressourcensparendes Verfahren erhalten wird, welches schnell auf neue/geänderte Behältnisinspektionsaufgaben anpassbar ist. Durch die in einem aufwändigen Maschinenlern-basierten Vor-Schritt erhaltenen Satz von extrahierten Merkmalen kann vorteilhaft zugleich eine hohe Inspektionsleistung erreicht werden.

Bei den extrahierten Behältnisteil-Merkmalen handelt es sich dabei insbesondere nicht um vorgegebene Merkmale, auch nicht um eine Auswahl (etwa von einem Nutzer) vorgegebener Merkmale. Bei den extrahierten Behältnisteil-Merkmalen handelt es sich insbesondere um abstrakte Merkmale, welche etwa einen Hell-Dunkel-Kontrast, eine für eine Häufigkeit von geraden Linien charakteristische Größe (etwa wie viele gerade Linien), eine Helligkeit oder einen Helligkeitsverlauf, Formen von Kontur(-Linien) und/oder Begrenzungslinien, Ecken, Formen, Anzahlen von Ecken, Krümmungen, Kombinationen hiervon und dergleichen angeben oder hierfür charakteristisch sind.

Bevorzugt sind die (insbesondere alle) Behältnisteil-Merkmale automatisch, bevorzugt im Rahmen des maschinellen Lernverfahrens, erzeugt (und insbesondere nicht ausgewählt).

Bevorzugt wird der Satz extrahierter Behältnisteil-Merkmale nicht angepasst und/oder verändert, auch nicht bei einer Festlegung einer (neuen und/oder weiteren und/oder anzupassenden) Behältnisinspektionsaufgabe.

Denkbar ist, dass eine Anzahl der Behältnisteil-Merkmale des Satzes extrahierter bzw. zu extrahierenden Behältnisteil-Merkmalen, etwa von einem Nutzer der Behältnisbehandlungsanlage, vorgegeben wird und/oder ist. Bevorzugt wird die vorgegebene Anzahl der Behältnisteil-Merkmale bei der Ermittlung des Satzes zu extrahierender Behältnisteil-Merkmale berücksichtigt. Diese vorgegebene Anzahl der Behältnisteil-Merkmale kann etwa von dem Nutzer der Behältnisbehandlungsanlage an einen externen Server, welcher die Ermittlung des Satzes zu extrahierender Behältnisteil-Merkmale durchführt, übermittelt werden. Denkbar ist aber auch, dass die Anzahl der Behältnisteil-Merkmale von einem Hersteller der Behältnisbehandlungsanlage vorgegeben ist/wird und insbesondere durch die Behältnisbehandlungsanlage (oder von einem Bediener dieser) nicht beeinflussbar ist.

Bevorzugt erfolgt eine Änderung und/oder Anpassung einer Behältnisinspektionsaufgabe und/oder einer Hinzufügung einer weiteren Behältnisinspektionsaufgabe ohne Änderung des Satzes extrahierter Behältnisteil-Merkmale, wobei die (weitere) Behältnisinspektionsaufgabe auf Grundlage des (unveränderten) Satzes extrahierter Behältnisteil-Merkmale durchgeführt wird.

Bevorzugt ist und/oder wird der Satz extrahierter Behältnisteil-Merkmale derart in der Behältnisinspektionsvorrichtung und/oder in der Echtzeit-Bildauswertungseinrichtung abgelegt, dass er (auch bei Änderung und/oder Hinzufügung einer Behältnisinspektionsaufgabe) nicht änderbar ist.

Bei der Behältnisinspektionsaufgabe handelt es sich insbesondere um eine Inspektionsaufgabe in Bezug auf ein Behältnisteil, insbesondere eine Klassifikationsaufgabe, beispielsweise eine Bild-Klassifikationsaufgabe, in Bezug auf ein Behältnisteil. Weitere mögliche Beispiele, bei welchen sich das vorgeschlagene Verfahren als vorteilhaft herausgestellt hat, sind in einem nachfolgenden Abschnitt angegeben.

Bevorzugt wird die Bild-Klassifikationsaufgabe durch eine Kombination der beiden im einleitenden Beschreibungsteil beschriebenen Verfahren gelöst. Die Merkmalsextraktion erfolgt dabei durch ein neuronales Netz, das mit umfangreichen Daten auf ähnliche Aufgaben der Bild-Klassifikation vortrainiert wurde. Im letzten Schritt, der Bewertung der extrahierten Merkmale wird dann jedoch ein klassisches Klassifikationsverfahren angewendet.

Das vorgeschlagene Verfahren bietet folgende Vorteile:
Ein klassisches Klassifikationsverfahren erlaubt eine schnelle Anpassung an neue Gegebenheiten innerhalb von Sekunden direkt auf der Maschine, und ohne umfangreiche Trainingsdaten. Eine Anpassung an eine neue Flaschensorte, kann so anhand von nur einem einzigen Bild der neuen Sorte gelernt werden.

Die aufwändig zu trainierende Merkmalsextraktion, bevorzugt mit Hilfe eines neuronalen Netzes, wird wiederverwendet, und braucht deshalb nicht mehr angepasst werden.

Bei einem bevorzugten Verfahren handelt es sich bei dem Satz von Behältnisteil-Merkmalen um einen im Rahmen eines überwachten Lernverfahrens Satz von extrahierten Behältnisteil-Merkmalen. Bevorzugt wird zur Durchführung des überwachten Lernverfahrens ein Satz von Trainingsdaten verwendet, welcher Sensordaten in Bezug auf Behältnisteile umfasst, welche mit einem in dem jeweiligen Fall zu erhaltenden Inspektionsergebnis der vorgegebenen Trainings-Behältnisinspektionsaufgabe gelabelt bzw. gekennzeichnet sind.

Bei einem weiter bevorzugten Verfahren handelt es sich bei dem überwachten Lernverfahren um einen K-nächste-Nachbarn-Algorithmus (englisch: "K nearest neighbour"-Algorithmus, auch abgekürzt als "k-NN" oder "KNN"). Bei diesem handelt es sich vorteilhaft um einen leicht an neu hinzugefügte Trainingsmuster anpassbaren und einfachen Algorithmus. Der K-nächste-Nachbarn-Algorithmus benötigt lediglich einen k-Wert und eine Distanzmetrik (Abstandsmetrik), was im Vergleich zu anderen Algorithmen im maschinellen Lernen wenig ist.

Bei einem weiter bevorzugten Verfahren wird (als Lernverfahren) ein traditioneller machine Learning Algorithmus (Maschinenlern-Algorithmus), wie beispielsweise Decision Trees (Decision Tree Learning, dt.: "Entscheidungsbaum-Lernen", wobei ein Entscheidungsbaum ein nichtparametrischer, überwachter Lernalgorithmus mit bevorzugt einer hierarchischen, baumartigen Struktur ist, der beispielsweise sowohl für Klassifizierungs- als auch für Regressionsaufgaben verwendet wird), Random Forests (deutsch: Zufallswald) oder Random Decision Forest, Logistic Regression (dt. logistische Regression), k-Means Clustering, Support Vector Machines (Abkürzung SVM, nicht-gebräuchliche dt. Übersetzung: "Stützvektormaschine" oder Stützvektormethode) verwendet.

Bei einem weiter bevorzugten Verfahren ist in der Echtzeit-Auswertungseinrichtung eine Vielzahl von weiteren (neuen und/oder angepassten), einander (insbesondere paarweise) verschiedenen Behältnisinspektionsaufgaben, insbesondere nutzerspezifisch, festlegbar und/oder vorgebbar, wobei jede dieser Behältnisinspektionsaufgaben auf Grundlage des (gleichen) der Echtzeit-Auswertungseinrichtung bereitgestellten und/oder vorgegebenen, in dem (insbesondere zur Trainings-Behältnisinspektionsaufgabe) maschinellen Lernverfahren, (extrahierten) Satzes von (extrahierten) Behältnisteil-Merkmalen durchgeführt wird.

Dies bietet den Vorteil, dass lediglich ein einziger Trainingsprozess bzw. ein einziges maschinelles Lernverfahren, nämlich der im Rahmen zur Trainings-Behältnisinspektionsaufgabe durchgeführte Trainingsprozess bzw. maschinelle Lernverfahren, durchgeführt wird. Die hier erhaltenen extrahierten Behältnisteil-Merkmale werden sodann für andere Behältnisinspektionsaufgaben verwendet.

Beispielsweise können die extrahierten Merkmalsvektoren einer Flasche in einem (Kamera- )Bild in mehreren Inspektionsaufgaben/ Klassifikationen verwendet werden.

Beispiel für zwei Inspektionsaufgaben/Klassifikationen:
1. Zeigen die Merkmalsvektoren eine braune Flasche?
2. Zeigen die Merkmalsvektoren eine verschlossene Flasche?

Beides ist in einem Bild und damit auch in dem extrahierten Merkmalsvektor erkennbar. Im ersten Fall wird der Algorithmus maschinellen Lernens, etwa kNN, dann mit Merkmalsvektoren von braunen und andersfarbigen Flaschen "trainiert". Im zweiten Fall mit Merkmalsvektoren von verschlossenen und unverschlossenen Flaschen.

Die Merkmale müssen nur einmal aus jedem Bild extrahiert werden.

Bei einem weiter bevorzugten Verfahren führt die Echtzeit-Auswertungseinrichtung, insbesondere bei einer vorgenommenen nutzerspezifischen Einstellung, die in Bezug auf jedes einzelne Behältnisteil erfassten Sensordaten sowohl die Trainings-Behältnisinspektionsaufgabe als auch die Behältnisinspektionsaufgabe durch.

Die Echtzeit-Auswertungseinrichtung kann also mit anderen Worten sowohl die Trainings-Behältnisinspektionsaufgabe durchführen, für welches ein maschinelles Lernverfahren durchgeführt wurde, als auch eine hiervon verschiedene Behältnisinspektionsaufgabe durchführen, welche nicht bei dem maschinellen Lernverfahren berücksichtigt wurde, deren Durchführung aber ebenfalls auf in dem maschinellen Lernverfahren erhaltenen Ergebnissen (nämlich dem Satz extrahierter Behältnisteil-Merkmale) basiert.

Bevorzugt ist von einem Bediener der Behältnisbehandlungsanlage einstellbar, ob das Trainings-Behältnisinspektionsverfahren und/oder das Behältnisinspektionsverfahren durchgeführt werden soll. Denkbar ist auch, dass beide Verfahren gleichzeitig durchgeführt werden (von der Echtzeit-Auswertungseinrichtung).

Bei einem weiter bevorzugten Verfahren wird als die durchzuführende Behältnisinspektionsaufgabe eine Klassifikationsaufgabe in Bezug auf, insbesondere genau, ein Referenz-Behältnisteil festgelegt, wobei der Echtzeit-Auswertungseinrichtung zur Festlegung der Klassifikationsaufgabe Referenz-Sensordaten zu dem Referenz-Behältnisteil bereitgestellt werden, wobei die Durchführung der (festgelegten) Klassifikationsaufgabe auf Grundlage des bereitgestellten Satzes von extrahierten Behältnisteil-Merkmalen erfolgt. Mit anderen Worten erfolgt eine Definition einer (neu/angepassten) Behältnisinspektionsaufgabe auf Grundlage des bereitgestellten Satzes von extrahierten Behältnisteil-Merkmalen. Hierdurch kann vorteilhaft eine sehr kurzfristige Festlegung einer Behältnisinspektionsaufgabe erfolgen.

Bei einem weiter bevorzugten Verfahren wird die durchzuführende Behältnisinspektionsaufgabe und/oder Klassifikationsaufgabe in Bezug auf Referenz-Sensordaten zu weniger als 100 Referenz-Behältnisteile, bevorzugt weniger als 50 Referenz-Behältnisteile und besonders bevorzugt weniger als 5 Referenz-Behältnisteile festgelegt. Dies bietet insbesondere im Vergleich zu einem aufwändigen Trainingsprozess bzw. maschinellen Lernverfahren, in welchen eine sehr hohe Anzahl an Sensordaten in Bezug auf Referenz-Behältnisteile erforderlich für eine hohe Inspektionsleistung der gelernten Behältnisinspektionsaufgabe sind, den Vorteil, dass hier nur Sensordaten in Bezug auf eine äußerst geringe Anzahl an Referenz-Behältnisteile erforderlich sind. Auch dies führt zu einer schnellen Einrichtung einer neuen Behältnisinspektionsaufgabe, etwa zur Erkennung eines neuen Flaschentyps.

Bei einem weiter bevorzugten Verfahren werden zur Festlegung der durchzuführenden Behältnisinspektionsaufgabe die Referenz-Sensordaten in Bezug auf das Referenz-Behältnisteil oder eine Vielzahl an Referenz-Sensordaten in Bezug auf eine Vielzahl von Referenz-Behältnisteile über eine Mensch-Maschine-Schnittstelle an die Echtzeit-Auswertungseinrichtung übermittelt. So kann etwa schnell ein Bild einer neuen Behältnissorte übermittelt werden, in Bezug auf welche die (neue) Behältnisinspektionsaufgabe auf Grundlage des Satzes extrahierter Behältnisteil-Merkmale durchzuführen ist.

Bei einem weiter bevorzugten Verfahren erfolgt die/eine Festlegung der durchzuführenden Behältnisinspektionsaufgabe, insbesondere unterbrechungsfrei, während einem laufenden Arbeitsbetrieb der Behältnisbehandlungsanlage und/oder Inspektionsbetrieb der Behältnisinspektionsvorrichtung.

Bei einem weiter bevorzugten Verfahren erfolgt die/eine Festlegung der durchzuführenden Behältnisinspektionsaufgabe ohne einen (weiteren) Trainingsschritt eines maschinellen Lernverfahrens.

Bei einem weiter bevorzugten Verfahren wird ein Merkmalsraum gebildet wird, welcher durch den bereitgestellten Satz von extrahierten Behältnisteil-Merkmalen aufgespannt wird.

Bevorzugt wird eine Distanzmetrik (Abstandsmetrik) in Bezug auf den Merkmalsraum bereitgestellt, wobei die Echtzeit-Auswertungseinrichtung die, insbesondere ortsaufgelösten, Sensordaten mittels der Distanzmetrik (Abstandsmetrik) auswertet.

Bevorzugt wird eine Distanzmetrik (Abstandsmetrik) in Bezug auf den Merkmalsraum bereitgestellt, wobei zusätzlich oder alternativ die Echtzeit-Auswertungseinrichtung die Distanzmetrik (Abstandsmetrik) als Ähnlichkeitsmaß zwischen, insbesondere ortsaufgelösten, Sensordaten verschiedener Behältnisteile und bevorzugt verschiedener Behältnisse und/oder als Ähnlichkeitsmaß zwischen erfassten Sensordaten und (etwa im Rahmen der Festlegung der Behältnisinspektionsaufgabe vorgegebener) Referenz-Sensordaten verwendet.

Bevorzugt wird (im Rahmen der Durchführung der Behältnisinspektionsaufgabe) eine Ähnlichkeit zwischen erfassten, insbesondere ortsaufgelösten, Sensordaten eines Behältnisteils mit denjenigen eines anderen Behältnisteils (etwa als Referenz-Sensordaten vorgegeben) mittels der Distanzmetrik beurteilt.

Bevorzugt wird zu allen erfassten (insbesondere ortsaufgelösten) Sensordaten, also beispielsweise für jedes aufgenommene Kamerabild, basierend auf dem Satz von extrahierten Behältnisteil-Merkmalen ein Merkmalsvektor erstellt. Bei dem Merkmalsvektor kann es sich beispielsweise um einen 256-dimensionalen Vektor handeln.

Bevorzugt weist der Merkmalsvektor höchstens 512 Dimensionen, bevorzugt höchstens 256 Dimensionen und besonders bevorzugt höchstens 128 Dimensionen auf. Bevorzugt weist der Merkmalsvektor mindestens 16, bevorzugt mindestens 32, bevorzugt mindestens 64 und besonders bevorzugt mindestens 128 Dimensionen auf. Grundsätzlich könnten aber auch Merkmalsvektoren mit mehr als 512 Dimensionen bzw. ein Merkmalsraum entsprechend höherer Dimensionalität verwendet werden.

Bevorzugt kann im Rahmen der Durchführung der Behältnisinspektionsaufgabe mittels der Distanzvektor der Abstand von des Merkmalsvektors von in Bezug auf ein erstes Behältnisteil erfassten Sensordaten und dem Merkmalsvektor von den in Bezug auf ein weiteres Behältnisteil erfassten Sensordaten ermittelt werden.

Bevorzugt kann im Rahmen der Durchführung der Behältnisinspektionsaufgabe mittels der Distanzvektor der Abstand von des Merkmalsvektors von in Bezug auf ein erstes Behältnisteil erfassten Sensordaten und einem zu Referenz-Sensordaten ermittelten Merkmalsvektor ermittelt werden.

Auf diese Weise können mittels der Distanzmetrik die Merkmalsvektoren (und damit die entsprechenden erfassten Sensordaten) ermittelt werden, welche am ähnlichsten zu einem vorgegebenen Merkmalsvektor waren/sind.

Bevorzugt werden Merkmalsvektoren (und damit die entsprechend erfassten Sensordaten) mit in Bezug auf die vorgegebene Distanzmetrik möglichst geringem Abstand zu einem vorgegebenen Merkmalsvektor ermittelt.

Denkbar ist aber auch, dass derjenige Merkmalsvektor oder diejenigen Merkmalsvektoren (und damit die entsprechend erfassten Sensordaten) ermittelt werden, welche eine in Bezug auf die vorgegebene Distanzmetrik einen möglichst hohen (bzw. den größten) Abstand zu vorgegebenen Merkmalsvektoren und/oder zu den Merkmalsvektoren bisher erfasster (oder in einem bestimmten Zeitraum) Sensordaten und/oder durchschnittlichen und/oder häufiger Merkmalsvektoren aufweisen. Auf diese Weise ist es möglich, sehr selten auftretende Sensordaten (etwa mit einem sehr selten auftretenden Defekt und/oder mit einer selten auftretenden Behältnisteilsorte) zu identifizieren.

Bei einem weiter bevorzugten Verfahren wird als Distanzmetrik eine euklidische Metrik und/oder eine Kosinus-Ähnlichkeit in dem Merkmalsraum verwendet. Die Kosinus-Ähnlichkeit (im engl. auch bekannt als "Cosine Similarity" oder "Cosine Distance") ist ein Maß für die Ähnlichkeit zweier Vektoren, bei welcher der Kosinus des Winkels zwischen beiden Vektoren bestimmt wird. Die Kosinus-Ähnlichkeit kann insbesondere als Maß verstanden werden, wie ausgeprägt zwei Vektoren in die gleiche Richtung zeigen. Die Kosinus-Ähnlichkeit zwischen zwei Vektoren a und b kann insbesondere berechnet werden aus dem Standardskalarprodukt aus den Vektoren a und b, dividiert durch die euklidische Norm aus a und die euklidische Norm aus b, also: Kosinus-Ähnlichkeit = (**a** · **b**)/(∥**a**∥ ∥**b**∥).

Bei einem weiter bevorzugten Verfahren handelt es sich bei der Behältnisinspektionsaufgabe um eine Klassifikationsaufgabe, welche ausgewählt ist aus einer Gruppe von Klassifikationsaufgaben, welche eine (bevorzugt binäre) Klassifikation in defekte und/oder defektfreie Behältnisteile und bevorzugt Behältnisse (gute / schlechte Behältnisse), eine Erkennung und/oder Klassifikation von Defektarten des Behältnisteils und bevorzugt des Behältnisses, eine Erkennung und/oder Klassifikation von verschiedenen Sorten des Behältnisteils und bevorzugt des Behältnisses (etwa, beispielsweise zehn, verschiedene Flaschentypen), eine Erkennung und/oder Klassifikation einer Kontur und/oder Farbe des Behältnisteils und bevorzugt des Behältnisses, eine Erkennung und/oder Klassifikation einer fehlerfreien und/oder fehlerbehafteten Durchführung wenigstens eines, an dem inspizierten Behältnisteil, insbesondere durch die (erste) Behandlungseinrichtung, vorgenommenen Behandlungsschritts, eine Identifizierung in dem Behältnisteil-Strom vergleichsweise selten auftretender Behältnisteil-Sorten und/oder Defektarten (selten meint insbesondere weniger als 1/1000), eine Etikettenkontrolle, eine Füllhöhenkontrolle, eine Überprüfung eines Schäumungsverhaltens einer Flüssigkeit in einem Behältnis, eine Erkennung eines Haarrisses und/oder Mündungsbruchs eines Behältnisses und/oder Bruchs in einem Bodenbereich des Behältnisses, eine Erkennung von in oder an dem Behältnisteil und/oder Behältnis angeordneten oder befindlichen Fremdpartikeln, und dergleichen sowie Kombinationen hiervon umfasst.

Bevorzugt handelt es sich bei dem Arbeitsbetrieb um einen laufenden (Produktions-)Betrieb der Behältnisinspektionsvorrichtung und/oder einen laufenden (Produktions-)Betrieb einer Behältnisbehandlungsanlage wie etwa einer Behältnisabfüllanlage, welche die Behältnisinspektionsvorrichtung aufweist. Insbesondere kann es sich bei dem Arbeitsbetrieb um einen Produktionsbetrieb handeln. Insbesondere handelt es sich bei dem Arbeitsbetrieb nicht um einen Testbetrieb und/oder Wartungsbetrieb und/oder Einstellbetrieb mit etwa gegenüber einer Transportgeschwindigkeit in einem Arbeitsbetrieb verringerten Transportgeschwindigkeit der Behältnisse bzw. Behältnisteile (bei ihrem Durchlauf durch die Behältnisinspektionsvorrichtung).

Bevorzugt ist die Sensoreinrichtung ausgewählt aus einer Gruppe, die eine Bildaufnahmeeinrichtung, etwa eine Kamera (bevorzugt Schwarz-weiß und/oder farbig), einen CMOS-Sensor (CMOS Abk. für Complementary metal-oxide-semiconductor, engl. für "komplementärer / sich ergänzender Metall-Oxid-Halbleiter), einen CCD-Sensor, einen 3D-Sensor, eine Bildaufnahmeeinrichtung auf Röntgen-Basis, ein optisches Element, eine Wärmebildkamera, eine Stereo-Kamera, eine LIDAR-Kamera, ein Geruchssensor und/oder ein (Massen-)Spektrometer, und dergleichen sowie Kombinationen umfasst.

In einer bevorzugten Ausführungsform transportiert die Transporteinrichtung die Behältnisse von einer ersten Behandlungseinrichtung zu einer weiteren (oder zweiten) Behandlungseinrichtung (und/oder ist insbesondere hierfür geeignet und bestimmt).

Bevorzugt ist die erste und/oder die weitere Behandlungseinrichtung ausgewählt aus einer Gruppe, welche eine Spritzgießvorrichtung zum Herstellen eines Spritzgießteils (etwa Vorformlings), eine Reinigungsvorrichtung zum Reinigen der Behältnisse und/oder Behältnisteile, eine Zerkleinerungseinrichtung zum Zerkleinern der Behältnisse, eine Füllvorrichtung zum Füllen der Behältnisse, eine Umformungsvorrichtung zum Umformen eines Kunststoffvorformlings in ein Kunststoffbehältnis, insbesondere eine Blasmaschine, eine Verschließeinrichtung zum Verschließen der Behältnisse, eine Etikettiervorrichtung, eine Markierungseinrichtung, eine Sortiereinrichtung, eine Verpackungseinrichtung, eine Bestimmungseinrichtung zur Bestimmung der Zusammensetzung von Stoffen oder Stoffgemischen, etwa der Atmosphäre und/oder Luft in oder an einem Behältnisteil und bevorzugt Behältnis, beispielsweise ein Massenspektrometer und/oder eine Geruchssensoreinrichtung, und dergleichen sowie Kombinationen hiervon umfasst.

Bevorzugt handelt es sich bei dem Behältnisteil-Strom um einen (insbesondere kontinuierlichen) Strom von (auf dem Transportpfad) sukzessiv bzw. aufeinanderfolgender Behältnisteilen. Beispielsweise kann es sich bei dem Behältnisteil-Strom um einen Behältnisstrom handeln, nämlich einem Strom von (auf dem Transportpfad) sukzessiv bzw. aufeinanderfolgenden Behältnissen. Dabei kann der Behältnisteil-Strom bereichsweise und bevorzugt innerhalb der gesamten Behältnisinspektionsvorrichtung (als Massenstrom) einbahnig oder aber auch mehrbahnig (mittels der Transporteinrichtung) geführt bzw. transportiert werden. Bevorzugt ist wenigstens je eine Sensoreinrichtung einer Bahn des Behältnisteil-Stroms zugeordnet und erfasst jedes auf dieser Bahn befindliche Behältnisteil des Behältnisteil-Stroms.

Bei der Transporteinrichtung kann es sich dabei auch um einen Massentransporteur zum, bevorzugten mehrbahnigen und/oder ungeordneten, Transport einer Vielzahl von Behältnisteilen und bevorzugt von Behältnissen handeln. Bei der Transporteinrichtung kann es sich dabei auch um einen Pufferbereich zum, bevorzugt mehrbahnigen und/oder ungeordneten, Puffern einer Vielzahl von Behältnisteilen und bevorzugt von Behältnissen handeln.

Die Behältnisteile und bevorzugt die Behältnisse können, bevorzugt wenigstens abschnittsweise und bevorzugt entlang in dem gesamten Transportbereich, stehend bzw. aufrecht (von der Transporteinrichtung) transportiert bzw. geführt werden.

Bevorzugt ist die Transporteinrichtung zum wenigstens abschnittsweisen Führen bzw. Transportieren der Vielzahl von Behältnisteilen und bevorzugt von Behältnissen, bevorzugt entlang des gesamten Transportbereichs, von unter Staudruck stehenden Behältnisteilen und bevorzugt Behältnissen geeignet und bestimmt.

Bevorzugt ist die Transporteinrichtung zum Transportieren und/oder Führen (zumindest innerhalb dem Transportbereich) von mindestens 1 Behältnisteil (und bevorzugt mindestens ein Behältnis) pro Stunde, bevorzugt mindestens 5000 (insbesondere zu inspizierende) Behältnisteile (und bevorzugt Behältnisse) pro Stunde, bevorzugt mindestens 20.000 (insbesondere zu inspizierende) Behältnisteile (und bevorzugt Behältnisse) pro Stunde, bevorzugt mindestens 100.000 (insbesondere zu inspizierende) Behältnisteile (und bevorzugt Behältnisse), bevorzugt mindestens 140.000 (insbesondere zu inspizierende) Behältnisteile (und bevorzugt Behältnisse) und besonders bevorzugt mindestens 180.000 (insbesondere zu inspizierende) Behältnisteile (und bevorzugt Behältnisse) geeignet und bestimmt und nimmt dies innerhalb des Arbeitsbetriebs der Behandlungseinrichtung und/oder Behältnisbehandlungsanlage vor. Bevorzugt ist die Transporteinrichtung zum Transportieren und/oder Führen (zumindest innerhalb dem Transportbereich) von höchstens 180.000 (besonders bevorzugt höchstens 200.000), insbesondere zu inspizierenden, Behältnisteilen (und bevorzugt Behältnissen) pro Stunde geeignet und bestimmt und nimmt dies innerhalb des Arbeitsbetriebs der Behandlungseinrichtung und/oder der Behältnisbehandlungsanlage und/oder der Behältnisinspektionsvorrichtung vor.

Bevorzugt ist die Transporteinrichtung in einem einbahnigen Transportbereich zum Transportieren und/oder Führen (zumindest innerhalb dem einbahnigen Transportbereich) von mindestens 100.000 Behältnisteilen (und bevorzugt Behältnissen) pro Stunde und/oder bis zu 180.000 (besonders bevorzugt höchstens 200.000) Behältnisteilen (und bevorzugt Behältnissen) pro Stunde geeignet und bestimmt und nimmt dies innerhalb des Arbeitsbetriebs der Behandlungseinrichtung und/oder der Behältnisbehandlungsanlage und/oder der Behältnisinspektionsvorrichtung vor.

Bei den Behältnissen kann es sich um Vorformlinge handeln, aus welchen durch einen Umformvorgang fertig ausgeformte Behältnisse hergestellt werden und/oder welche bisher nur durch einen Urformschritt hergestellt sind. Bei den Behältnissen kann es sich auch um bereits fertig ausgeformte Behältnisse handeln, welche beispielsweise durch einen Umformvorgang eines Vorformlings (etwa eines Spritzgussteils bzw. eines Spritzlings) ihre fertige Ausformung erreicht haben.

Bei den Behältnissen kann es sich um leere, noch zu befüllende und/oder zu recycelnde und/oder wieder zu befüllende Behältnisse handeln. Bei den Behältnissen kann es sich auch um befüllte Behältnisse handeln. Zusätzlich oder alternativ kann es sich bei den Behältnissen um mit (insbesondere genau) einem Behältnisverschluss verschlossene und/oder verschließbare Behältnisse handeln.

Bei den Behältnissen kann es sich um Einwegbehältnisse oder um Mehrwegbehältnissen handeln.

Bei den Behältnissen handelt es sich bevorzugt um (bevorzugt dichte), insbesondere verschließbare, Behältnisse zur Aufnahme von Flüssigkeiten und/oder fließfähigen Substanzen, etwa pastöse und/oder cremeartige und/oder gelartige Substanzen, wie etwa aus dem Lebensmittelbereich, aus der Kosmetikindustrie oder aus dem pharmazeutischen Bereich.

Denkbar ist auch, dass es sich bei den Behältnissen um Behältnisse zur Aufnahme von Flüssigkeiten und/oder Körpern handelt, wie etwa für Behältnisse zur Aufnahme von Kontaktlinsen.

Bevorzugt handelt es sich bei der externen Speichereinrichtung um eine Cloud-basierte (bevorzugt nicht-flüchtigen) Speichereinrichtung und/oder einen externen Server (inkl. Speichereinrichtung), wobei auf die Speichereinrichtung insbesondere über das Internet (und/oder über ein, insbesondere wenigstens abschnittsweise drahtgebundenes und/oder drahtloses, öffentliches und/oder privates Netzwerk) zugegriffen wird. Unter einem externen Server ist insbesondere ein in Bezug auf eine Behältnisinspektionsvorrichtung und/oder Echtzeit-Auswertungseinrichtung und/oder Einstelleinrichtung externer Server, insbesondere ein Backend-Server, zu verstehen.

Der externe Server ist beispielsweise ein Backend insbesondere eines Behältnisinspektionsvorrichtungs-Herstellers oder eines Dienstanbieters, welcher dazu eingerichtet ist, ortsaufgelöste Sensordaten (insbesondere einer Vielzahl von Sensoreinrichtungen und/oder einer Vielzahl von Behältnisinspektionsvorrichtungen) zu verwalten und/oder maschinelle Lernverfahren in Bezug auf durchzuführende (Trainings-)Behältnisinspektionsverfahren durchzuführen und/oder Behältnisinspektionsvorrichtungen einzustellen und/oder anzupassen. Die Funktionen des Backend bzw. des externen Servers können dabei auf (externen) Serverfarmen durchgeführt werden. Beim (externen) Server kann es sich um ein verteiltes System handeln.

Die vorliegende Erfindung ist weiterhin gerichtet auf eine Behältnisinspektionsvorrichtung, für eine Behältnisbehandlungsanlage zur Behandlung einer Vielzahl von Behältnisteilen für Behältnisse und bevorzugt für Kunststoffbehältnisse und/oder Flaschen. Dabei dient die Behältnisinspektionsvorrichtung zur Durchführung einer Behältnisinspektionsaufgabe in der Behältnisbehandlungsanlage bzw. ist hierfür geeignet und bestimmt.

Dabei ist in der Behältnisbehandlungsanlage eine Transporteinrichtung vorgesehen zum Transport der Vielzahl von Behältnisteilen als Behältnisteil-Strom entlang eines vorgegebenen Transportpfads von wenigstens einer Behandlungseinrichtung der Behältnisbehandlungsanlage zu wenigstens einer weiteren Behandlungseinrichtung der Behältnisbehandlungsanlage.

Dabei weist die Behältnisinspektionsvorrichtung wenigstens eine Sensoreinrichtung zur Durchführung der Behältnisinspektionsaufgabe, welche zur, bevorzugt optischen, Erfassung, insbesondere ortsaufgelöster, Sensordaten und bevorzugt Kamerabilder in Bezug auf die zu inspizierenden Behältnisteile, geeignet und bestimmt ist, auf.

Weiter weist die Behältnisinspektionsvorrichtung eine Echtzeit-Auswertungseinrichtung zur Auswertung der, insbesondere ortsaufgelösten, Sensordaten und bevorzugt Kamerabilder, in Echtzeit auf.

Erfindungsgemäß ist der Echtzeit-Auswertungseinrichtung ein Satz von Behältnisteil-Merkmalen vorgegeben und/oder vorgebbar, bei welchem es sich um einen Satz von, im Rahmen eines in Bezug auf eine Trainings-Behältnisinspektionsaufgabe durchgeführten maschinellen Lernverfahrens automatisch, insbesondere von einem neuronalen Netz, extrahierten Behältnisteil-Merkmalen handelt. Dabei ist bevorzugt die Trainings-Behältnisinspektionsaufgabe von der Behältnisinspektionsaufgabe verschieden. Die Behältnisteil-Merkmale werden bevorzugt bei einem maschinellen Lernverfahren bzw. bei Vornahme eines maschinellen Lernverfahrens extrahiert, welches in Bezug auf eine Trainings-Behältnisinspektionsaufgabe durchgeführt wird. Mit der Durchführung des maschinellen Lernverfahrens soll ein (trainierter) Algorithmus bzw. ein (trainiertes) Modell (maschinellen Lernens) erhalten werden, welches zur Erfüllung bzw. zur Durchführung der Trainings-Behältnisinspektionsaufgabe dient.

Bevorzugt handelt es sich bei dem Satz von extrahierten Behältnisteil-Merkmalen um einen, im Rahmen des in Bezug auf die Trainings-Behältnisinspektionsaufgabe durchgeführten maschinellen Lernverfahrens (automatisch) extrahierten Satz von (extrahierten) Behältnisteil-Merkmalen.

Weiter ist die Echtzeit-Auswertungseinrichtung zur Durchführung der Behältnisinspektionsaufgabe basierend auf dem vorgegebenen Satz von Behältnisteil-Merkmalen geeignet, bestimmt und/oder konfiguriert ist.

Es wird also auch im Rahmen der erfindungsgemäßen vorgeschlagen, dass der Echtzeit-Auswertungseinrichtung mit dem bereitgestellten (extrahierten) Satz von extrahierten Behältnisteil-Merkmalen Kl-basierte, aber zugleich flexibel anpassbare Auswertungsmittel zur Auswertung von Sensordaten zur Verfügung gestellt werden.

Bevorzugt ist die Behältnisinspektionsvorrichtung dazu eingerichtet, geeignet und/oder bestimmt, dass obig beschriebene Verfahren zur Durchführung einer Behältnisinspektionsaufgabe sowie alle bereits obig im Zusammenhang mit dem Verfahren beschriebene Verfahrensschritte einzeln oder in Kombination miteinander auszuführen. Umgekehrt kann das Verfahren mit allen im Rahmen der Behältnisinspektionsvorrichtung beschriebenen Merkmalen einzeln oder in Kombination miteinander ausgestattet sein.

Die vorliegende Erfindung ist weiter gerichtet auf ein, insbesondere Computer-implementiertes, Verfahren zur Ermittlung, insbesondere zur Merkmalsextraktion, eines Satzes von Behältnisteil-Merkmalen zur Verwendung in einer Behältnisinspektionsvorrichtung zur Durchführung einer Behältnisinspektionsaufgabe, umfassend die Schritte:
- Bereitstellen einer Trainings-Behältnisinspektionsaufgabe;
- Bereitstellen eines Trainigsdatensatzes umfassend eine Vielzahl von Sensordaten in Bezug auf eine Vielzahl von Behältnisteilen für Behältnisse und umfassend jeweils ein Label, welches ein beabsichtigtes Ergebnis der Trainings-Behältnisinspektionsaufgabe angibt;
- Durchführen eines, bevorzugt überwachten, maschinellen Lernverfahrens auf Grundlage des Trainingsdatensatzes in Hinblick auf die Trainings-Behältnisinspektionsaufgabe;
- Extraktion der in dem maschinellen Lernverfahren erhaltenen Behältnisteil-Merkmale. Die Merkmalsextraktion kann von dem Algorithmus des maschinellen Lernverfahrens als erste Verarbeitungsstufe durchgeführt werden. Sie stellt eine vorteilhafte Möglichkeit dar, die Verarbeitungseffizienz deutlich zu verbessern und den Einfluss irrelevanter Informationen zu reduzieren. Dabei wird die Merkmalsextraktion automatisch während der Trainingsphase des maschinellen Lernverfahrens gelernt.

Das maschinelle Lernverfahren kann dabei von einem (insbesondere tiefen) neuronalen Netzwerk, etwa einem "Convolutional Neural Networks" (CNN) durchgeführt werden. Diese lernen etwa während des gesamten Trainingsprozesses (etwa in einem Computer-Vision-Verfahren als Behältnisinspektionsaufgabe) automatisch, aus (Roh-)Sensordaten sinnvolle Merkmale wie Kanten, Formen und Texturen zu extrahieren.

Bei einem bevorzugten Verfahren werden in dem Trainingsprozess bzw. dem maschinellen Lernverfahren Trainingsdaten verwendet, welche eine von der wenigstens einen Sensoreinrichtung erfasste Vielzahl von, insbesondere ortsaufgelösten, Sensordaten (von Behältnissen) umfassen. Dies bietet den Vorteil, dass bereits der Trainingsprozess spezifisch auf die einzustellende Behältnisinspektionsvorrichtung abgestimmt wird und beispielsweise damit spezifische Gegebenheiten der spezifischen Behältnisinspektionsvorrichtung, wie optische Eigenschaften der Sensoreinrichtung oder auch spezifische Lichtverhältnisse in der Behältnisinspektionsvorrichtung, unmittelbar berücksichtigt werden können.

Bevorzugt sind/werden die zur Verwendung als Trainingsdaten vorgesehenen (von der wenigstens einen Sensoreinrichtung erfassten), insbesondere ortsaufgelösten, Sensordaten mit (Behältnis-)Sorten- und/oder Klassifikationsmerkmalen (je nach Klassifikationsaufgabe, etwa Klassifikation der Defektart) versehen. Bevorzugt werden die, insbesondere ortsaufgelösten, Sensordaten zusammen mit den jeweils ihnen zugeordneten (Behältnis-)Sorten- und/oder Klassifikationsmerkmalen als Trainingsdatensatz (insbesondere auf der externen und/oder der nicht-flüchtigen Speichereinrichtung) abgelegt. Bevorzugt wird auf diese Weise eine Vielzahl von Trainings-Datensätzen erzeugt. Bei den Klassifikationsmerkmalen kann es sich dabei um die Klassen der Trainings-Behältnisinspektionsaufgabe (bzw. Ergebnisklassen zu den obig beschriebenen Behältnisinspektionsaufgaben) handeln. So können etwa die einem Behältnisteil zugeordneten, insbesondere ortsaufgelösten, Sensordaten mit den darin vorkommenden Defektarten und dergleichen klassifiziert sein.

Bei der nicht-flüchtigen Speichereinrichtung kann es sich um eine Speichereinrichtung handeln, welche (fester) Bestandteil der wenigstens einen Sensoreinrichtung und/oder der Behältnisinspektionsvorrichtung ist. Denkbar ist, dass es sich bei dieser Speichereinrichtung um einen Ringspeicher handelt, bei welchem bei Erreichen der Speicherkapazität die ältesten Sensordaten überschrieben werden (und somit die abgelegten Sensordaten nur für einen begrenzten Zeitraum zur Verfügung stehen).

Bei der nicht-flüchtigen Speichereinrichtung kann es sich ebenfalls um eine Speichereinrichtung der Behältnisbehandlungsanlage handeln, welche etwa als Festspeicher ausgebildet ist.

"Nicht-flüchtig" kann auch heißen, dass die selektierten Bilder bzw. Sensordaten und/oder die auf der nicht-flüchtigen Speichereinrichtung gespeicherten Sensordaten nur über einen gewissen Zeitraum gehalten werden, also beispielsweise nach einer einstellbaren Zeit oder Parametrieraktion (Monteur) gelöscht werden (können). "Nicht-flüchtig" kann auch heißen, dass die Bilder bzw. Sensordaten nicht über das Ausschalten der Maschine hinaus gehalten werden bzw. gespeichert bleiben müssen.

"Nicht-flüchtig" soll auch bedeuten, dass die zu "haltenden" Bilddaten und/oder die abzulegenden Sensordaten im einfachsten Fall für eine Parametrierung zur Verfügung stehen müssen.

Denkbar ist auch, dass zusätzlich oder alternativ unter "nicht-flüchtiger Speichereinrichtung" verstanden wird, dass die zu "haltenden" Bilddaten und/oder die abzulegenden Sensordaten auch erhalten bleiben, wenn die Speichereinrichtung nicht mit Strom versorgt ist.

Das Trainieren der Netze erfordert eine Anzahl von markierten und/oder klassifizierten Sensordaten (beispielsweise Bilder) pro Anwendung der Größenordnung von 1.000 bis 100.000 (beispielsweise 10.000 markierte und/oder klassifizierte Bilder pro Anwendung). Diese Markierung und/oder Klassifizierung kann lokal oder zentral von Bildverarbeitungs-Experten erfolgen.

Bevorzugt ist (zusätzlich oder alternativ), dass als Trainingsdaten, (bevorzugt ausschließlich) insbesondere ortsaufgelöste, Sensordaten von Behältnisteilen (oder hiervon abgeleitete Daten) verwendet werden, die von einer Sensoreinrichtung (wenigstens) einer anderen, bevorzugt baugleichen, Behältnisinspektionsvorrichtung (bevorzugt desselben Herstellers) erfasst wurden. Dies bietet den Vorteil, dass hierdurch eine große Vielzahl an Sensordaten noch vor einer Inbetriebnahme der Behältnisinspektionsanlage bereitgestellt und verwendet werden kann.

Denkbar ist auch, dass als Trainingsdaten (ausschließlich oder teilweise) synthetisch erzeugte oder via Augmentierung (engl. data augmentation) erzeugte ortsaufgelöste Sensordaten (oder hiervon abgeleitete Daten) verwendet werden. Dies bietet den Vorteil, dass etwa selten auftretende Klassen von Defektarten hierdurch simuliert und das Modell maschinellen Lernens hiermit in effizienter Weise trainiert werden kann.

Bevorzugt erfolgt das Training bzw. das maschinelle Lernverfahren mittels überwachtem Lernen. Es wäre jedoch auch möglich, das maschinelle Lernverfahren mittels unbeaufsichtigtem Lernen, bestärkendem Lernen oder stochastischem Lernen zu trainieren.

Bevorzugt werden die extrahierten Behältnisteil-Merkmale einer Behältnisinspektionsvorrichtung bzw. einer Echtzeit-Auswertungseinrichtung einer Behältnisinspektionsvorrichtung (wie obig beschrieben) bereitgestellt und/oder übermittelt.

Bevorzugt wird das Verfahren an einem in Bezug auf die Behältnisbehandlungsanlage externen Server durchgeführt und/oder der Satz extrahierter Behältnisteil-Merkmale auf einem in Bezug auf die Behältnisbehandlungsanlage externen Speichereinrichtung abgelegt. Bevorzugt ist der abgelegte Satz extrahierter Behältnisteil-Merkmale (nach Freigabe) von dem Betreiber der Behältnisbehandlungsanlage abrufbar.

Bei einem vorteilhaften Verfahren wird die Anzahl der zu extrahierenden Behältnisteilmerkmale des Satzes von Behältnisteil-Merkmalen vorgegeben.

Der Trainingsprozess bzw. das maschinelle Lernverfahren kann dabei lokal (bei der Behältnisinspektionsvorrichtung) und/oder zentral und/oder örtlich unabhängig und/oder auf einem externen Server in Bezug auf die Behältnisinspektionsvorrichtung und/oder die Behältnisbehandlungsanlage vorgenommen werden.

Bei einem vorteilhaften Verfahren wird das maschinelle Lernverfahren und/oder die Merkmalsextraktion örtlich getrennt von der Behältnisbehandlungsanlage durchgeführt, insbesondere außerhalb eines Betriebsgeländes der Behältnisbehandlungsanlage.

Die vorliegende Erfindung ist weiterhin gerichtet auf eine Behältnisbehandlungsanlage zum Behandeln von Behältnisteilen für Behältnisse umfassend eine Behältnisinspektionsvorrichtung gemäß einer obig beschriebenen (bevorzugten) Ausführungsform und umfassend eine Behandlungseinrichtung, wenigstens eine weitere Behandlungseinrichtung und eine Transporteinrichtung zum Transportieren der Behältnisteile von der Behandlungseinrichtung zur wenigstens einen weiteren Behandlungseinrichtung.

Bevorzugt ist die Behältnisbehandlungsanlage dazu eingerichtet, geeignet und/oder bestimmt, das obig beschriebene Verfahren zur Durchführung einer Behältnisinspektionsaufgabe sowie alle bereits obig im Zusammenhang mit dem Verfahren beschriebene Verfahrensschritte einzeln oder in Kombination miteinander auszuführen. Weiter kann die Behältnisbehandlungsanlage und/oder die Behandlungseinrichtung und/oder die wenigstens eine weitere Behandlungseinrichtung und/oder die Transporteinrichtung wenigstens ein obig beschriebenes Merkmal einzeln oder in Kombination mit weiteren Merkmalen aufweisen bzw. ausgestattet sein.

Die vorliegende Erfindung ist weiterhin gerichtet auf ein Computerprogramm oder Computerprogrammprodukt, umfassend Programmmittel, insbesondere einen Programmcode, welcher zumindest einige der und bevorzugt alle Verfahrensschritte des erfindungsgemäßen Verfahrens und bevorzugt eine der beschriebenen bevorzugten Ausführungsformen repräsentiert oder kodiert und zum Ausführen durch eine Prozessoreinrichtung ausgebildet ist.

Die vorliegende Erfindung ist weiterhin gerichtet auf einen Datenspeicher, auf welchem zumindest eine Ausführungsform des erfindungsgemäßen Computerprogramms oder eine bevorzugte Ausführungsform des Computerprogramms gespeichert ist.

Die vorliegende Erfindung wurde in Bezug auf ein Behältnis bzw. Behältnisteile für Behältnisse beschrieben. Dabei ist die vorliegende Erfindung auch übertragbar auf Spritzgussteile (Spritzlinge) oder allgemein auf in einer Behandlungsanlage zu behandelnde Artikel (etwa herzustellende und/oder zu verpackende Kontaktlinsen), deren Behandlungsfortschritt und/oder deren Eigenschaften und/oder (Defekt-/Qualitäts-)Zustände durch wenigstens eine Sensoreinrichtungen (zur Erfassung von insbesondere ortsaufgelösten Sensordaten in Bezug auf jeden einzelnen zu inspizierenden Artikel) kontrolliert wird. Die Anmelderin behält sich vor, hierauf gerichtete Gegenstände ebenfalls zu beanspruchen.

Die vorliegende Erfindung ist weiterhin gerichtet auf ein Verfahren zur Durchführung einer Artikelinspektionsaufgabe in einer Artikelbehandlungsanlage zur Behandlung einer Vielzahl von Spritzgussteilen und/oder Artikel, in welcher eine Transporteinrichtung, die Vielzahl von Spritzgussteile und/oder Artikel als Artikel-Strom entlang eines vorgegebenen Transportpfads von wenigstens einer Behandlungseinrichtung der Artikelbehandlungsanlage zu wenigstens einer weiteren Behandlungseinrichtung der Artikelbehandlungsanlage transportiert und wenigstens eine Sensoreinrichtung zur Durchführung der Artikelinspektionsaufgabe insbesondere ortsaufgelöste Sensordaten, bevorzugt Kamerabilder, in Bezug auf die zu inspizierenden Spritzgussteile und/oder Artikel, bevorzugt optisch, erfasst und eine Echtzeit-Auswertungseinrichtung die insbesondere ortsaufgelösten Sensordaten, bevorzugt die Kamerabilder, in Echtzeit auswertet.

Erfindungsgemäß ist der Echtzeit-Auswertungseinrichtung ein Satz von Artikel-Merkmalen vorgegeben, bei welchem es sich um einen Satz von, im Rahmen eines in Bezug auf eine von der Artikelinspektionsaufgabe verschiedenen Trainings-Artikelinspektionsaufgabe durchgeführten maschinellen Lernverfahrens automatisch, insbesondere von einem neuronalen Netz, extrahierten Artikel-Merkmalen handelt. Dabei führt die Echtzeit-Auswertungseinrichtung die Artikelinspektionsaufgabe basierend auf dem vorgegebenen Satz von Artikel-Merkmalen durch.

Die vorliegende Erfindung ist weiterhin gerichtet auf ein Verfahren zur Ermittlung, insbesondere zur Merkmalsextraktion, eines Satzes von Artikel-Merkmalen zur Verwendung in einer Artikelinspektionsvorrichtung zur Durchführung einer Artikelinspektionsaufgabe, umfassend die Schritte:
- Bereitstellen einer Trainings-Artikelinspektionsaufgabe;
- Bereitstellen eines Trainingsdatensatzes umfassend eine Vielzahl von Sensordaten in Bezug auf eine Vielzahl von Spritzgussteile und/oder Artikel und umfassend jeweils ein Label, welches ein beabsichtigtes Ergebnis der Trainings-Artikelinspektionsaufgabe angibt;
- Durchführen eines, bevorzugt überwachten, maschinellen Lernverfahrens auf Grundlage des Trainingsdatensatzes in Hinblick auf die Trainings-Artikelinspektionsaufgabe;
- Extraktion der in dem maschinellen Lernverfahren erhaltenen Artikel-Merkmale.

Die vorliegende Erfindung ist weiterhin gerichtet auf eine Artikelinspektionsvorrichtung, für eine Artikelbehandlungsanlage zur Behandlung einer Vielzahl von Spritzgussteilen und/oder Artikel, zur Durchführung einer Artikelinspektionsaufgabe in der Artikelbehandlungsanlage, wobei in der Artikelbehandlungsanlage eine Transporteinrichtung vorgesehen ist zum Transport der Vielzahl von Spritzgussteile und/oder Artikel als Artikel-Strom entlang eines vorgegebenen Transportpfads von wenigstens einer Behandlungseinrichtung der Artikelbehandlungsanlage zu wenigstens einer weiteren Behandlungseinrichtung der Artikelbehandlungsanlage, und wobei die Artikelinspektionsvorrichtung wenigstens eine Sensoreinrichtung zur Durchführung der Artikelinspektionsaufgabe, welche zur, bevorzugt optischen, Erfassung, insbesondere ortsaufgelöster, Sensordaten und bevorzugt Kamerabilder in Bezug auf die zu inspizierenden Spritzgussteile und/oder Artikel, geeignet und bestimmt ist, und eine Echtzeit-Auswertungseinrichtung zur Auswertung der, insbesondere ortsaufgelösten, Sensordaten und bevorzugt Kamerabilder, in Echtzeit aufweist.

Erfindungsgemäß ist der Echtzeit-Auswertungseinrichtung ein Satz von Artikel-Merkmalen

vorgegeben und/oder vorgebbar, bei welchem es sich um einen Satz von, im Rahmen eines in Bezug auf eine von der Artikelinspektionsaufgabe verschiedenen Trainings-Artikelinspektionsaufgabe durchgeführten maschinellen Lernverfahrens automatisch, insbesondere von einem neuronalen Netz, extrahierten Artikel-Merkmalen handelt. Dabei ist die Echtzeit-Auswertungseinrichtung zur Durchführung der Artikelinspektionsaufgabe basierend auf dem vorgegebenen Satz von Artikel-Merkmalen geeignet und bestimmt.

Die weiteren obig im Rahmen der Behältnisteile beschriebenen Merkmale sind entsprechend analog auf die Spritzgussteile und/oder Artikel anwendbar.

Weitere Vorteile und Ausführungsformen ergeben sich aus der beigefügten Zeichnung:
Darin zeigen:
- Fig. 1: eine schematische Darstellung einer erfindungsgemäßen Behältnisbehandlungsanlage gemäß einer bevorzugten Ausführungsform; und
- Fig. 2: Kamerabilder zur Veranschaulichung des erfindungsgemäßen Verfahrens gemäß einer bevorzugten Ausführungsform.

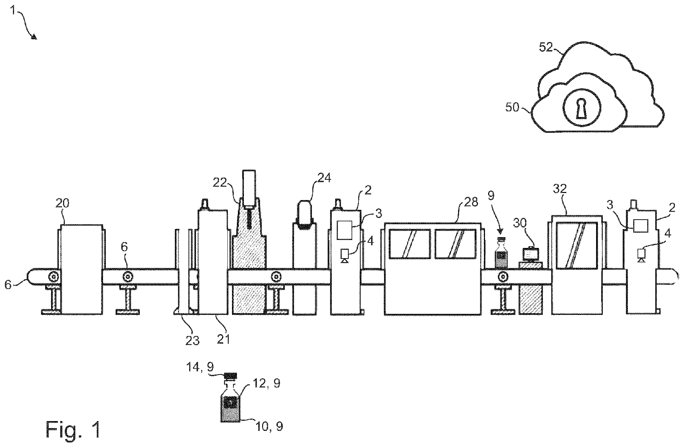

Fig. 1 zeigt eine schematische Darstellung einer erfindungsgemäßen Behältnisbehandlungsanlage gemäß einer bevorzugten Ausführungsform.

Fig. 1 zeigt eine schematische Darstellung einer erfindungsgemäßen Behältnisbehandlungsanlage 1 zum Behandeln von Behältnisteilen 10, hier als Flaschen ausgebildete Behältnisse 10, gemäß einer ersten Ausführungsform.

Das Bezugszeichen 12 kennzeichnet einer an dem Behältnisteil 10, hier einem Behältnis, angeordneten Ausstattung. In dem in Fig. 1 dargestellten Ausführungsbeispiel ist als Ausstattung ein Identifikationsmittel beispielhaft gezeigt, welches an der Flasche 10 angeordnet ist. Dabei handelt es sich beispielsweise um einen (aufgedruckten) QR-Code. Das Bezugszeichen 14 kennzeichnet einen Behältnisverschluss als eine weitere Ausstattung des Behältnisses 10.

In der in Fig. 1 gezeigten Ausführungsform wird ein Kunststoffvorformling bereitgestellt und von der Transporteinrichtung 6 einer Erwärmungsvorrichtung 20 zugeführt, in dieser erwärmt und im Anschluss in einer Blasformvorrichtung als weitere Behandlungseinrichtung, deren Anordnung innerhalb der Behältnisbehandlungsanlage 1 durch das Bezugszeichen 23 gekennzeichnet ist, zu einer (Kunststoff-)Flasche 10 expandiert. Diese Flasche 10 kann beispielsweise durch die Individualisierungseinrichtung, etwa einer Druckeinrichtung, mit einem Identifikationsmittel 12 versehen sein, wodurch eine Flasche, welche ein Identifikationsmittel 12 aufweist, entsteht.

Das Behältnisteil 9 kann in der Behältnisbehandlungsanlage 1 von wenigstens einer Transporteinrichtung 6 von einer Behandlungseinrichtung zur nächsten, sowie auch innerhalb der Behandlungseinrichtung(en) transportiert werden. Dargestellt sind hier als Behandlungseinrichtungen (in einer Reihenfolge stromabwärts der Transportrichtung der Flasche) eine Inspektionsvorrichtung 21, eine Füllvorrichtung 22 zum Befüllen der Flasche 10 mit einem Produkt, eine Verschließvorrichtung 24, eine Trocknungsvorrichtung 28, eine Etikettiervorrichtung 30 sowie eine Verpackungseinrichtung 32 zum Verpacken der Flasche 10.

Die Bezugszeichen 2 kennzeichnet jeweils eine weitere Behältnisinspektionsvorrichtung (etwa am Ende der Linie und zwischen der Verschließeinrichtung 24 und der Trocknungseinrichtung 28 angeordnet), welche beispielsweise eine Füllhöhe in der Flasche und/oder eine ordnungsgemäße Anordnung des Verschlusses auf der Flasche 10 und/oder einen Sicherungsring und/oder eine ordnungsgemäße Etikettierung und/oder Verpackung der Flasche 10 oder weitere Produktionsdaten überprüft.

Das Bezugszeichen 4 kennzeichnet jeweils eine Sensoreinrichtung, hier einer Kamera, mittels welcher - individuell zu jedem (zu inspizierenden) Behältnisteil 9 - Sensordaten in Bezug auf das jeweilige Behältnisteil 9 erhoben bzw. erfasst bzw. aufgenommen werden.

Das Bezugszeichen 3 kennzeichnet jeweils eine Echtzeit-Auswertungseinrichtung, mittels welcher die von der Sensoreinrichtung 4 der jeweiligen Behältnisinspektionsvorrichtung 2 erfassten Sensordaten zur Durchführung einer (vorgegebenen und/oder festgelegten) Behältnisinspektionsaufgabe ausgewertet werden.

Bei dem vorgeschlagenen Verfahren wird zur Auswertung ein Satz extrahierter Merkmale zur Bewertung der erfassten Sensordaten verwendet. Der verwendete Satz extrahierter Merkmale ist das Ergebnis einer (trainierten) Merkmalsextraktion, welche durch ein neuronales Netz, das mit (umfangreichen) (Trainings-)Daten auf ähnliche (Inspektions-)Aufgaben der Bild-Klassifikation vortrainiert wurde. Im letzten Schritt der Bewertung der extrahierten Merkmale wird dann jedoch ein klassisches Klassifikationsverfahren angewendet.

Das Bezugszeichen 50 kennzeichnet einen internen Server bzw. eine interne Speichereinrichtung und das Bezugszeichen 52 einen externen Server bzw. eine externe, insbesondere Cloud-basierte, Speichereinrichtung. Auf dem externen Server 52 kann beispielsweise die Al-basierte Merkmalsextraktion vorgenommen werden. Auf der externen und/oder der internen Speichereinrichtung 50/52 kann bevorzugt der erhaltene Satz extrahierter Behältnisteil-Merkmale abgelegt werden.

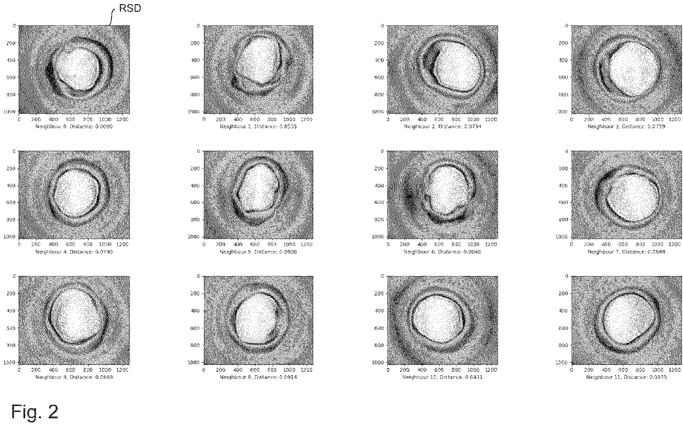

Figur 2 zeigt zwölf Kamerabilder zur Veranschaulichung des erfindungsgemäßen Verfahrens gemäß einer bevorzugten Ausführungsform. Diese Kamerabilder werden im Rahmen einer Bodeninspektion eines Behältnisses von einer Kamera aufgenommen, welche durch die Mündung des Behältnisses hindurch den Boden des Behältnisses inspiziert. Dabei wird der Boden in einem Durchlichtverfahren von einer Beleuchtungseinrichtung beleuchtet.

Das erste Bild, in der Figurenebene der Fig. 2 links oben, welches mit dem Bezugszeichen RSD gekennzeichnet ist, wird dabei als Referenz-Bild verwendet. Dieses weist somit den mittels (etwa einer euklidischen) Abstandsmetrik ermittelten Abstand 0 zu sich selbst auf.

Die weiteren in Fig. 2 gezeigten Kamerabilder sind entsprechend dem jeweiligen in Bezug auf die Abstandsmetrik ermittelten Abstand zu dem Referenz-Bild sortiert (von links nach rechts, dann von oben nach unten), und zeigen damit einen steigenden Abstand. Das letzte Kamerabild, in der Figurenebene rechts unten angeordnet, hat den im Vergleich zu den anderen in Fig. 2 gezeigten Kamerabildern höchsten Abstand mit einem Abstand von 0,0955 und ist somit der elfte Nachbar des Referenz-Bildes.

Die Ähnlichkeit der Kamerabilder beruht dabei auf einer starken bzw. weniger starken Ausprägung eines Vorhandenseins von (Rest-)Flüssigkeit in dem jeweils inspizierten Behältnis.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind. Es wird weiterhin darauf hingewiesen, dass in den einzelnen Figuren auch Merkmale beschrieben wurden, welche für sich genommen vorteilhaft sein können. Der Fachmann erkennt unmittelbar, dass ein bestimmtes in einer Figur beschriebenes Merkmal auch ohne die Übernahme weiterer Merkmale aus dieser Figur vorteilhaft sein kann. Ferner erkennt der Fachmann, dass sich auch Vorteile durch eine Kombination mehrerer in einzelnen oder in unterschiedlichen Figuren gezeigter Merkmale ergeben können.

### Bezugszeichenliste

- 1: Behältnisbehandlungsanlage
- 2, 21: Behältnisinspektionsvorrichtung
- 3: Echtzeit-Auswertungseinrichtung
- 4: Sensoreinrichtung
- 6: Transporteinrichtung
- 10: Behältnis
- 9: Behältnisteil
- 12: Ausstattung, Direktdruckelement
- 14: Ausstattung, Behältnisverschluss
- 20: Behandlungseinrichtung, hier Erwärmungseinrichtung
- 23: Behandlungseinrichtung, hier Druckeinrichtung
- 22: Behandlungseinrichtung, hier Fülleinrichtung
- 24: Behandlungseinrichtung, hier Verschließeinrichtung
- 28: Behandlungseinrichtung, hier Trocknungseinrichtung
- 30: Behandlungseinrichtung, hier Etikettiereinrichtung
- 32: Behandlungseinrichtung, hier Verpackungseinrichtung
- 50: interner Server, Speichereinrichtung
- 52: externer Server Speichereinrichtung

## Patentansprüche

1. Verfahren zur Durchführung einer Behältnisinspektionsaufgabe in einer Behältnisbehandlungsanlage (1) zur Behandlung einer Vielzahl von Behältnisteilen (9) für Behältnisse (10), bevorzugt Kunststoffbehältnisse und/oder Flaschen, in welcher eine Transporteinrichtung (6), die Vielzahl von Behältnisteilen (9) als Behältnisteil-Strom entlang eines vorgegebenen Transportpfads von wenigstens einer Behandlungseinrichtung (20, 23, 22, 24, 28, 30) der Behältnisbehandlungsanlage (1) zu wenigstens einer weiteren Behandlungseinrichtung (23, 22, 24, 28, 30, 32) der Behältnisbehandlungsanlage (1) transportiert und wenigstens eine Sensoreinrichtung (4) zur Durchführung der Behältnisinspektionsaufgabe insbesondere ortsaufgelöste Sensordaten, bevorzugt Kamerabilder, in Bezug auf die zu inspizierenden Behältnisteile (9), bevorzugt optisch, erfasst und eine Echtzeit-Auswertungseinrichtung (3) die insbesondere ortsaufgelösten Sensordaten, bevorzugt die Kamerabilder, in Echtzeit auswertet, **dadurch gekennzeichnet, dass**
der Echtzeit-Auswertungseinrichtung (3) ein Satz von Behältnisteil-Merkmalen vorgegeben ist, bei welchem es sich um einen Satz von, im Rahmen eines in Bezug auf eine von der Behältnisinspektionsaufgabe verschiedenen Trainings-Behältnisinspektionsaufgabe durchgeführten maschinellen Lernverfahrens automatisch, insbesondere von einem neuronalen Netz, extrahierten Behältnisteil-Merkmalen handelt, und dass die Echtzeit-Auswertungseinrichtung (3) die Behältnisinspektionsaufgabe basierend auf dem vorgegebenen Satz von Behältnisteil-Merkmalen durchführt.

2. Verfahren nach Anspruch 1, wobei es sich bei dem Satz von Behältnisteil-Merkmalen um einen im Rahmen eines überwachten Lernverfahrens Satz von extrahierten Behältnisteil-Merkmalen handelt.

3. Verfahren nach einem der vorangegangenen Ansprüche, **dadurch gekennzeichnet, dass** es sich bei dem überwachten Lernverfahren um einen K-nächste-Nachbarn-AIgorithmus handelt.

4. Verfahren nach einem der vorangegangenen Ansprüche, **dadurch gekennzeichnet, dass** in der Echtzeit-Auswertungseinrichtung eine Vielzahl von weiteren, einander verschiedenen Behältnisinspektionsaufgaben, insbesondere nutzerspezifisch, festlegbar und/oder vorgebbar ist, wobei jede dieser Behältnisinspektionsaufgabe auf Grundlage des, der Echtzeit-Auswertungseinrichtung bereitgestellten Satzes von extrahierten Behältnisteil-Merkmalen durchgeführt wird.

5. Verfahren nach einem der vorangegangenen Ansprüche, **dadurch gekennzeichnet, dass** die Echtzeit-Auswertungseinrichtung, insbesondere bei einer vorgenommenen nutzerspezifischen Einstellung, die in Bezug auf jedes einzelne Behältnisteil erfassten Sensordaten sowohl die Trainings-Behältnisinspektionsaufgabe als auch die Behältnisinspektionsaufgabe durchführt.

6. Verfahren nach einem der vorangegangenen Ansprüche, **dadurch gekennzeichnet, dass** als die durchzuführende Behältnisinspektionsaufgabe eine Klassifikationsaufgabe in Bezug auf, insbesondere genau, ein Referenz-Behältnisteil festgelegt wird, wobei der Echtzeit-Auswertungseinrichtung (3) zur Festlegung der Klassifikationsaufgabe Referenz-Sensordaten (RSD) zu dem Referenz-Behältnisteil bereitgestellt werden, wobei die Durchführung der festgelegten Klassifikationsaufgabe auf Grundlage des bereitgestellten Satzes von extrahierten Behältnisteil-Merkmalen erfolgt.

7. Verfahren nach einem der vorhergehenden Ansprüche, **dadurch gekennzeichnet, dass** die durchzuführende Behältnisinspektionsaufgabe und/oder Klassifikationsaufgabe in Bezug auf Referenz-Sensordaten (RSD) zu weniger als 100 Referenz-Behältnisteile, bevorzugt weniger als 50 Referenz-Behältnisteile und besonders bevorzugt weniger als 5 Referenz-Behältnisteile festgelegt wird.

8. Verfahren nach einem der beiden vorhergehenden Ansprüche, **dadurch gekennzeichnet, dass** zur Festlegung der durchzuführenden Behältnisinspektionsaufgabe die Referenz-Sensordaten (RSD) in Bezug auf das Referenz-Behältnisteil oder eine Vielzahl an Referenz-Sensordaten (RSD) in Bezug auf eine Vielzahl von Referenz-Behältnisteile über eine Mensch-Maschine-Schnittstelle an die Echtzeit-Auswertungseinrichtung (3) übermittelt werden.

9. Verfahren nach einem der vorangegangenen Ansprüche, **dadurch gekennzeichnet, dass** eine Festlegung der durchzuführenden Behältnisinspektionsaufgabe, insbesondere unterbrechungsfrei, während einem laufenden Arbeitsbetrieb der Behältnisbehandlungsanlage erfolgt und/oder dass eine Festlegung der durchzuführenden Behältnisinspektionsaufgabe ohne einen (weiteren) Trainingsschritt eines maschinellen Lernverfahrens erfolgt.

10. Verfahren nach einem der vorangegangenen Ansprüche, **dadurch gekennzeichnet, dass** ein Merkmalsraum gebildet wird, welcher durch den bereitgestellten Satz von extrahierten Behältnisteil-Merkmalen aufgespannt wird, und eine Distanzmetrik in Bezug auf den Merkmalsraum bereitgestellt wird, wobei die Echtzeit-Auswertungseinrichtung (3) die, insbesondere ortsaufgelösten, Sensordaten mittels der Distanzmetrik auswertet und/oder die Distanzmetrik als Ähnlichkeitsmaß zwischen, insbesondere ortsaufgelösten, Sensordaten verschiedener Behältnisteile und bevorzugt verschiedener Behältnisse verwendet.

11. Verfahren nach dem vorangegangenen Anspruch, **dadurch gekennzeichnet, dass** als Distanzmetrik eine euklidische Metrik und/oder eine Kosinus-Ähnlichkeit in dem Merkmalsraum verwendet wird.

12. Verfahren nach einem der vorangegangenen Ansprüche, **dadurch gekennzeichnet, dass** es sich bei der Behältnisinspektionsaufgabe um eine Klassifikationsaufgabe handelt, welche ausgewählt ist aus einer Gruppe von Klassifikationsaufgaben, welche eine Klassifikation in defekte und/oder defektfreie Behältnisteile und bevorzugt Behältnisse (gute / schlechte Behältnisse), eine Erkennung und/oder Klassifikation von Defektarten des Behältnisteils und bevorzugt des Behältnisses, eine Erkennung und/oder Klassifikation von verschiedenen Sorten des Behältnisteils und bevorzugt des Behältnisses (etwa, beispielsweise zehn, verschiedene Flaschentypen), eine Erkennung und/oder Klassifikation einer Kontur und/oder Farbe des Behältnisteils und bevorzugt des Behältnisses, eine Erkennung und/oder Klassifikation einer fehlerfreien und/oder fehlerbehafteten Durchführung wenigstens eines, an dem inspizierten Behältnisteil, insbesondere durch die (erste) Behandlungseinrichtung, vorgenommenen Behandlungsschritts, und dergleichen sowie Kombinationen hiervon umfasst.

13. Verfahren zur Ermittlung, insbesondere zur Merkmalsextraktion, eines Satzes von Behältnisteil-Merkmalen zur Verwendung in einer Behältnisinspektionsvorrichtung zur Durchführung einer Behältnisinspektionsaufgabe, umfassend die Schritte:
- Bereitstellen einer Trainings-Behältnisinspektionsaufgabe;
- Bereitstellen eines Trainigsdatensatzes umfassend eine Vielzahl von Sensordaten in Bezug auf eine Vielzahl von Behältnisteilen für Behältnisse und umfassend jeweils ein Label, welches ein beabsichtigtes Ergebnis der Trainings-Behältnisinspektionsaufgabe angibt;
- Durchführen eines, bevorzugt überwachten, maschinellen Lernverfahrens auf Grundlage des Trainingsdatensatzes in Hinblick auf die Trainings-Behältnisinspektionsaufgabe;
- Extraktion der in dem maschinellen Lernverfahren erhaltenen Behältnisteil-Merkmale.

14. Verfahren nach dem vorangegangenen Anspruch, **dadurch gekennzeichnet, dass** eine Anzahl der zu extrahierenden Behältnisteilmerkmale des Satzes von Behältnisteil-Merkmalen vorgegeben wird.

15. Behältnisinspektionsvorrichtung (2), für eine Behältnisbehandlungsanlage (1) zur Behandlung einer Vielzahl von Behältnisteilen (9) für Behältnisse (10) und bevorzugt für Kunststoffbehältnisse und/oder Flaschen, zur Durchführung einer Behältnisinspektionsaufgabe in der Behältnisbehandlungsanlage (1), wobei in der Behältnisbehandlungsanlage (1) eine Transporteinrichtung (6) vorgesehen ist zum Transport der Vielzahl von Behältnisteilen (9) als Behältnisteil-Strom entlang eines vorgegebenen Transportpfads von wenigstens einer Behandlungseinrichtung (20, 23, 22, 24, 28, 30) der Behältnisbehandlungsanlage (1) zu wenigstens einer weiteren Behandlungseinrichtung (23, 22, 24, 28, 30, 32) der Behältnisbehandlungsanlage (1), und wobei die Behältnisinspektionsvorrichtung (2) wenigstens eine Sensoreinrichtung (4) zur Durchführung der Behältnisinspektionsaufgabe, welche zur, bevorzugt optischen, Erfassung, insbesondere ortsaufgelöster, Sensordaten und bevorzugt Kamerabilder in Bezug auf die zu inspizierenden Behältnisteile (9), geeignet und bestimmt ist, und eine Echtzeit-Auswertungseinrichtung (3) zur Auswertung der, insbesondere ortsaufgelösten, Sensordaten und bevorzugt Kamerabilder, in Echtzeit aufweist,
**dadurch gekennzeichnet, dass**
der Echtzeit-Auswertungseinrichtung (3) ein Satz von Behältnisteil-Merkmalen vorgegeben und/oder vorgebbar ist, bei welchem es sich um einen Satz von, im Rahmen eines in Bezug auf eine von der Behältnisinspektionsaufgabe verschiedenen Trainings-Behältnisinspektionsaufgabe durchgeführten maschinellen Lernverfahrens automatisch, insbesondere von einem neuronalen Netz, extrahierten Behältnisteil-Merkmalen handelt, und dass die Echtzeit-Auswertungseinrichtung (3) zur Durchführung der Behältnisinspektionsaufgabe basierend auf dem vorgegebenen Satz von Behältnisteil-Merkmalen geeignet und bestimmt ist.
